(19) Europäisches Patentamt
European Patent Office
Office européen des brevets

(11) EP 1 197 214 A1

(12) # EUROPEAN PATENT APPLICATION
published in accordance with Art. 158(3) EPC

(43) Date of publication:
**17.04.2002 Bulletin 2002/16**

(21) Application number: **00942374.0**

(22) Date of filing: **28.06.2000**

(51) Int Cl.7: **A61K 31/7012**, A61P 1/12
// C07H7:02

(86) International application number:
**PCT/JP00/04264**

(87) International publication number:
**WO 01/00217 (04.01.2001 Gazette 2001/01)**

(84) Designated Contracting States:
**AT BE CH CY DE DK ES FI FR GB GR IE IT LI LU MC NL PT SE**
Designated Extension States:
**AL LT LV MK RO SI**

(30) Priority: **28.06.1999 JP 18232799**

(71) Applicants:
- **ZAIDAN HOJIN BISEIBUTSU KAGAKU KENKYU KAI**
**Shinagawa-ku, Tokyo 141-0021 (JP)**
- **Meiji Seika Kaisha, Ltd.**
**Tokyo 104-8002 (JP)**
- **NIPPON KAYAKU CO., LTD.**
**Chiyoda-ku, Tokyo 102-8172 (JP)**

(72) Inventors:
- **TAKEUCHI, Tomio**
**Shinagawa-ku Tokyo 141-0022 (JP)**
- **MURAOKA, Yasuhiko**
**Tokyo 175-0082 (JP)**
- **NISHIMURA, Yoshio**
**Tokyo 201-0005 (JP)**
- **TERAYAMA, Yoshihiko Pharm. Techn. Labo**
**Odawara-shi Kanagawa 250-0852 (JP)**
- **OKADA, Mineaki**
**Tokyo 115-0042 (JP)**
- **AKIYAMA, Yuji**
**Ohmiya-shi Saitama 330-0835 (JP)**
- **EKIMOTO, Hisao**
**Tokyo 115-0042 (JP)**

(74) Representative: **Guerre, Dominique et al**
**Cabinet Germain et Maureau,**
**12, rue Boileau,**
**BP 6153**
**69466 Lyon Cedex 06 (FR)**

(54) **MEDICINAL COMPOSITIONS FOR PREVENTING OR TREATING DIARRHEA**

(57) Provided is a pharmaceutical composition for the prevention or treatment of a diarrhea, which comprises as an active ingredient, siastatin B or a siastatin B derivative having a β-glucuronidase inhibitory activity. Siastatin B or the siastatin B derivative is useful as a drug for the prevention or treatment of a diarrhea, which is capable of ameliorating the diarrhea disorders as induced as a side effect when a cancer-bearing patient received the administration of an anticancer chemotherapeutic agent, particularly an anticancer camptothecin derivative acting as a topoisomerase I inhibitor. This composition is effective to prevent or treat such diarrhea as induced by the administration of an anticancer camptothecin derivative to the cancer-bearing patient.

EP 1 197 214 A1

## Description

### TECHNICAL FIELD

This invention relates to a new pharmaceutical composition for preventing or therapeutically treating a diarrhea as induced in a cancer-bearing patient having received a chemotherapy for the cancer. This invention relates particularly to a new pharmaceutical composition for preventing or treating a diarrhea disorder which has been induced in a cancer-bearing patient as a side effect that is associated with the administration of an anticancer chemotherapeutic agent. This invention further relates to a therapeutic method for preventing or ameliorating a diarrhea as induced in a cancer-bearing patient having received the administration of an anticancer chemotherapeutic agent.

### BACKGROUND ART

There are often observed diarrheaes which have been induced in patients due to the side effect of a medicine which was administered to the patients for the chemotherapy. In particular, such a diarrhea which has been induced in cancer-bearing patients due to the side effect of the administered anticancer chemotherapeutic agent is often severe. In some cases, such severe diarrhea can result in a necessity to discontinue the chemotherapeutic treatment for the cancer of the patients. Besides, such serious diarrhea as induced by the administration of the anticancer chemotherapeutic agent can deteriorate physical strength and nutritive conditions of the patients, which can further retard patient's recovery from the disordered conditions that the populations of leukocytes and blood platelets have been decreased by the administration of the anticancer chemotherapeutic agent. Particularly, when an anticancer camptothecin derivative, one of the anticancer chemotherapeutics agents which act as a topoisomerase I inhibitor is administered, it is frequent that the diarrhea as induced by the side effect of the administered anticancer agent become very much serious.

Generally speaking, many unclarities still remain to be elucidated for the mechanism of the incidence of diarrhea. In cases of the diarrhea which can be induced by the administered anticancer chemotherapeutic agent, however, it may be deduced that the diarrhea is induced due to the direct actions of the administered anticancer agent to disturb the mucous membrane of the gastrointestinal tracts, involving the biochemical change in the mucous membrane cells and also involving the change in the intestinal microflora. There has not yet been established any effective therapeutical method which can ameliorate such diarrhea as induced in association with the administration of the anticancer chemotherapeutic agent. Up to now, amelioration of the diarrhea has been attended by trying modification of the administration methods and/or doses of the anticancer chemotherapeutic agent, and improvement in the formulation of the anticancer pharmaceutical preparations as well as chemical modification of compounds to be used as such anticancer chemotherapeutic agent.

### DISCLOSURE OF THE INVENTION

A primary object of this invention is to provide a pharmaceutical composition for preventing or therapeutically treating diarrhea, which is capable of ameliorating the disorders such as diarrhea as induced as a side-effect in the cancer-bearing patients when the patients have received the administration of an anticancer chemotherapeutic agent, particularly such diarrhea as induced by the administration of an anticancer camptothecin derivative that acts as a topoisomerase I inhibitor. A second object of this invention is to provide a therapeutic method for ameliorating a diarrhea as induced in the cancer-bearing patient by the administration of an anticancer chemotherapeutic agent.

We, the inventors of this invention, have eagerly carried out our investigations in order to achieve the objects of this invention above-mentioned. As a result, we have now found that, when there is administered orally to the cancer-bearing patient siastatin B or a derivative of siastatin B which is already known, and which is represented by a general formula (I) given hereinafter and has an inhibitory activity against β-glucuronidase, before the time of the administration of the anticancer camptothecin derivative to the patient, siastatin B and the siastatin B derivative are effective to ameliorate the diarrhea as induced as the side effect by the administration of said camptothecin derivative. This invention has been completed on the basis of these findings.

According to a first aspect of this invention, therefore, there is provided a pharmaceutical composition for preventing or treating a diarrhea, which comprises as an active ingredient, siastatin B or a siastatin B derivative that has an inhibitory activity against β-glucuronidase and that is represented by the following general formula (I)

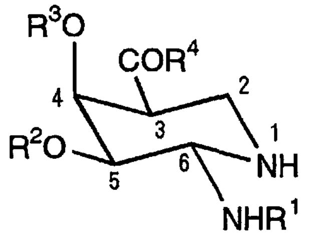

(I)

wherein $R^1$ is a hydrogen atom or an unsubstituted or halogen-substituted, straight or branched acyl group of 1-5 carbon atoms, particularly an alkanoyl group, or $R^1$ is a group of formula $-C(=NH)R^5$ in which $R^5$ is a group $-OR^6$ or $R^5$ is a group $-NR^7R^8$ where $R^6$, $R^7$ and $R^8$ each stand for a hydrogen-atom or a straight or branched alkyl group of 1-5 carbon atoms; and $R^2$ and $R^3$ may be the same or different from each other and $R^2$ and $R^3$ each are a hydrogen atom or an unsubstituted or halogen-substituted, straight or branched acyl group of 1-5 carbon atoms, particularly an alkanoyl group; and independently from $R^5$, $R^4$ stands for a group of formula $-OR^6$ or a group of formula $-NR^7R^8$ where $R^6$, $R^7$ and $R^8$ each stand for a hydrogen atom or a straight or branched alkyl group of 1-5 carbon atoms, or a pharmacologically acceptable salt thereof, in combination with a pharmacologically acceptable carrier or carriers.

Siastatin B itself is a compound of the general formula (I) above, wherein $R^1$ is acetyl group, $R^2$ and $R^3$ each are hydrogen atom and $R^4$ is -OH group [refer to J. Antibiot., Vol. 27, pp.963-969 (1974)].

In the pharmaceutical composition according to the first aspect of this invention, the pharmacologically acceptable salts of siastatin B or the siastatin B derivative, which is usable as the active ingredient, may include alkali metal salts, e.g. sodium salt, at the 3-carboxyl group of siastatin B, and alkaline earth metal salts, e.g. calcium salt, at said 3-carboxyl group, and ammonium salt, and so on, as well as acid addition salts at the imino group of siastatin B or the siastatin B derivative, with a pharmacologically acceptable inorganic acid, for example, hydrochloride, sulfate and phosphate, and with a pharmacologically acceptable organic acid, for example, acetate and para-toluenesulfonate. However, the salts are not limited thereto.

The pharmaceutical composition according to the first aspect of this invention for preventing or treating the diarrhea may preferably be administered to such cancer-bearing patients who have the diarrhea as induced as the side effect in association with the administration of the anticancer chemotherapeutic agent. In particular, the composition of this invention may suitably be administered for the prevention or treatment of a diarrhea which is induced in association with the administration of an anticancer chemotherapeutic agent of such type that a metabolic product of the administered anticancer agent can be converted in vivo into the form of a glucuronide thereof which is then excreted into the bile. As a particularly preferred application, the pharmaceutical composition according to the first aspect of invention is used to prevent or treat such diarrhea which is induced in the cancer-bearing patients by administration of a topoisomerase I inhibitor acting as the anticancer chemotherapeutic agent.

It is known that the topoisomerase I inhibitor usable as the anticancer chemotherapeutic agent includes camptothecin-related drugs which are namely the anticancer camptothecin derivatives. One example of the anticancer camptothecin derivatives is irinotecan, and further examples thereof are topotecan and others [refer to literature: J. Clin. Oncol., Vol. 10, page 647 (1992)]. It is already known that irinotecan, topotecan and the other camptothecin derivatives, after their administration to the patient, are metabolized in the liver of the patient so as to convert into the metabolite of the derivatives which is in the form of a glucuronide and which is then excreted in the bile and further transferred into the lower intestinal tracts, i.e. small intestine and large intestine of the patients.

According to our investigations, it is presumed that the mechanism of suppression of the diarrhea by administering siastatin B or the siastatin B derivative of the general formula (I) which has an inhibitory activity against β-glucuronidase comprises the following mechanism. That is, the camptothecin derivatives can be converted, after the administration thereof, into their metabolites in vivo by the liver, and the resulting metabolites are further converted by the action of UDP-glucuronosyl-transferase (UGT) existing in the liver, to produce glucuronides thereof which are thereafter excreted into the bile. There exist many sorts of enterobacteria of the intestinal microflora in the lower intestinal tracts, and some enterobacteria of them have β-glucuronidase. The bacteria having β-glucuronidase can decompose said metabolites of the camptothecin derivatives which have been converted into the glucuronides, so that the glucuronides are deconjugated into deglucuronyl product and glucuronic acid. As a result, it is presumed that such deglucuronyl product which have been regenerated in the intestines (which product is namely such aglycon-moiety compound as derived from the original camptothecin derivative) can act in the intestines so as to cause the diarrhea which is induced by the resulting cytopathy in the intestines [refer to Xenobiotica, Vol. 23, pp.5-10 (1993)].

Siastatin B and the siastatin B derivative of the general formula (I) to be used as the active ingredient in the composition of this invention are known, as explained hereinafter, to have a potent inhibitory activity against β-glucuronidase. This enzyme-inhibitory activity of siastatin B or its derivative can inhibit that the glucuronides are deconjugated

by β-glucuronidase within the intestines and hence that the above-mentioned deglucuronyl product is reproduced. In this way, siastatin B or the siastatin B derivative of the formula (I) can exhibit the inhibitory effect against the diarrhea. It is therefore believed that siastatin B or the siastatin B derivative of the formula (I) having the inhibitory activity against β-glucuronidase, after being orally administered and having arrived at the intestines, is able to inhibit such deconjugation of the said glucuronides which would be caused by the intestinal microflora bacteria having β-glucuronidase, and that siastatin B or the siastatin B derivative having the inhibitory activity against β-glucuronidase is thus able to inhibit the reproduction of the deglucuronyl product which would cause the diarrhea, whereby siastatin B or the siastatin B derivative is able to exert the preventive effect against the incidence of diarrhea and also is able to ameliorate the symptom of the diarrhea as caused.

Siastatin B or the siastatin B derivative of the general formula (I), which is to be used as the active ingredient in the composition of this invention, may be those compounds of the general formula (I) where $R^1$, $R^2$ and/or $R^3$ is or each are an unsubstituted or halogen-substituted, straight or branched acyl group of 1-5 carbon atoms. They may particularly be such siastatin B derivative of the general formula (I) wherein $R^1$, $R^2$ and/or $R^3$ is or each are a halogen-substituted or unsubstituted, straight or branched ($C_1$-$C_5$) alkanoyl group.

In cases where $R^1$, $R^2$ and/or $R^3$ in the general formula (I) above is an unsubstituted or halogen-substituted, ($C_1$-$C_5$) alkanoyl group, this alkanoyl group may preferably be, for example, formyl group, acetyl group, propionyl group, butyryl group, isobutyryl group, valeryl group, isovaleryl group, 2-methylbutyryl group, 2,2-dimethylpropionyl group, fluoroacetyl group, difluoroacetyl group, trifluoroacetyl group, chloroacetyl group, dichloroacetyl group, trichloroacetyl group, bromoacetyl group, iodoacetyl group, pentafluoropropionyl group, pentachloropropionyl group, heptafluorobutyryl group or heptachloroisobutyryl group, and the like.

In cases where $R^1$ in the formula (I) is a group -C(=NH)$R^5$, with $R^6$, $R^7$ and/or $R^8$ present in the group $R^5$ being an alkyl group of 1-5 carbon atoms, this ($C_1$~$C_5$) alkyl group may include methyl group, ethyl group, n-propyl group, i-propyl group, n-butyl group, i-butyl group, sec-butyl group, tert-butyl group, n-pentyl group, i-amyl group and tert-amyl group, for example. Further, when $R^4$ in the formula (I) is a group -O$R^6$, the alkyl group $R^6$ may be one of those alkyl groups as exemplified above.

Among the compounds of the general formula (I), particularly preferred examples thereof are such a siastatin B derivative of the formula (I) wherein $R^1$ is hydrogen atom, acetyl group, trifluoroacetyl group or trichloroacetyl group, or $R^1$ is amidino group -C(=NH)$NH_2$; and $R^2$ and $R^3$ each are hydrogen atom; and $R^4$ is a group -OH.

Siastatin B or the siastatin B derivative of the general formula (I), which is to be used as the active ingredient in the composition of this invention, may preferably be such siastatin B derivative of the following general formula (I')

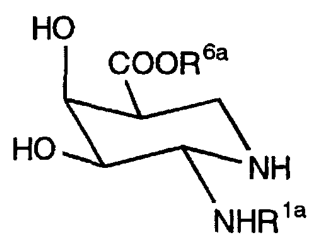

(I')

wherein $R^{1a}$ is hydrogen atom or a ($C_1$-$C_5$) alkanoyl group, or $R^{1a}$ is a ($C_1$-$C_5$) alkanoyl group which is mono-, di- or trisubstituted with chlorine or fluorine atom, or $R^{1a}$ is amidino group of formula -C(=NH)$NH_2$; and $R^{6a}$ is hydrogen atom or a ($C_1$-$C_5$) alkyl group, or a pharmacologically acceptable salt thereof.

Some specific examples of the compounds to be preferably used as the active ingredient in the composition of this invention are listed below.

(1) Siastatin B represented by the following formula (Ia)

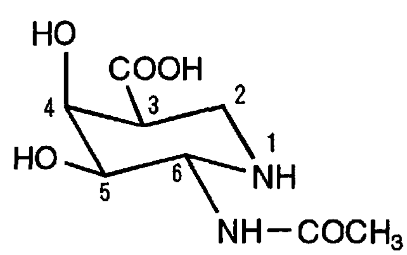

(Ia)

(2) 6-Deacetamido-6-trifluoroacetamido-siastatin B represented by the following formula (Ib)

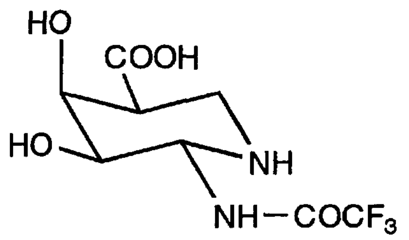

(Ib)

which is the compound given in Japanese Patent Application First Publication KOKAI Hei-6-157458 under the name of (3S,4S, 5R,6R)-6-(trifluoroacetamido)-4,5-dihydroxypiperidine-3-carboxylic acid.

(3) 6-Deacetamido-6-trichloroacetamido-siastatin B represented by the following formula (Ic)

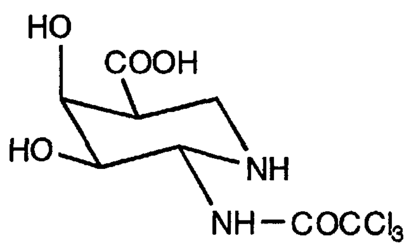

(Ic)

which is the compound given in Japanese Patent Application First Publication KOKAI Hei-9-71565 under the name of (3S,4S, 5R,6R)-6-(trichloroacetamido)-4,5-dihydroxypiperidine-3-carboxylic acid.

(4) 6-Deacetamido-6-guanidino-siastatin B represented by the following formula (Id)

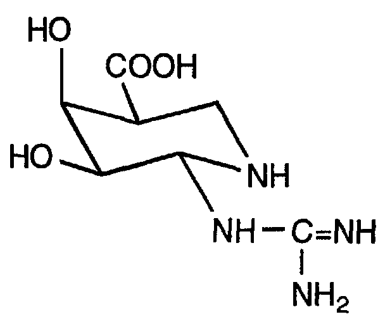

(Id)

which is the compound given in Japanese Patent Application First Publication KOKAI Hei-9-71565 under the name of (3S,4S, 5R,6R)-6-guanidino-4,5-dihydroxypiperidine-3-carboxylic acid.

(5) 6-Deacetamido-6-amino-siastatin B represented by the following formula (Ie)

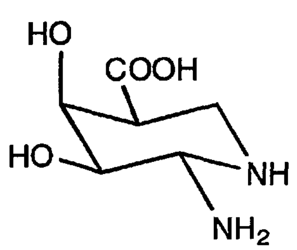

(Ie)

which is the reaction product as obtained by de-trifluoroacetylation of 6-deacetamido-6-trifluoroacetamidosiastatin

B of formula (Ib) by treating with hydrochloric acid in methanol.

Incidentally, the fact that each of the compounds of formula (Ia), formula (Ib), formula (Ic) and formula (Id) as shown above exhibit an inhibitory activity against β-glucuronidase is described in "J. Antibiotics" Vol. 47, pp.101-107 (1994) and ibid. Vol. 49, pp.321-325 (1996).

Siastatin B is one of the compounds of the general formula (I) which may to be used as the active ingredient in the composition of this invention. Siastatin B is known to be isolated as a fermentation product of strains of Actinomycetes or other strains from the culture broth thereof [refer, for example, to Japanese Patent Publication KOKAI Sho-55-46714 and "J. Antibiot." Vol. 27, pp.963-969 (1974)]. Further, siastatin B may be produced either by a total chemical synthesis or by semi-chemical synthesis with starting from sugars [See, for example, Japanese Patent Application First Publication KOKAI Hei-1-294687, "Bull. Chem. Soc. Jpn." 65, p.978 (1992) and "J. Am. Chem. Soc." 110, p.7249 (1988)].

All the siastatin B derivatives of the general formula (I) to be used as the active ingredient in the composition of this invention may be produced by known chemical conversion processes with starting from siastatin B. Excepting the compound of the general formula (I) wherein $R^1$ =acetyl group, $R^2$ =$R^3$ =H and $R^4$ =OH, that is, siastatin B itself, all the siastatin B derivatives of the general formula (I) may be produced from siastatin B by known processes, for example, by the process described in Japanese Patent Application First Publication KOKAI Hei-6-157458 and KOKAI Hei-9-71565, or by a process or processes comprising some combinations of steps for conventional protection and de-protection of functional groups of siastatin B, and steps for acylation or esterification or amidation reactions and so on. Further, such siastatin B derivatives of the formula (I) may also be produced directly by chemical synthetic processes without using siastatin B.

The pharmaceutical composition according to the first aspect of this invention contains a pharmaceutically acceptable carrier or carriers, in admixture with the active ingredient, and the composition may be formulated in the form of different preparations which have been conventionally used in the art. As the pharmaceutically acceptable carriers, there may be used a wide variety of solid carriers which have been used conventionally in usual pharmaceutical preparations, for example, starch, saccharose and lactose, as well as a variety of liquid carriers, which may be, for example, water, physiological saline solution, or ethanol and aqueous ethanol. If desired, the composition of this invention may contain also excipient, binder, disintegrator, lubricant, colorant, flavoring agent, smell improver and/or surfactant in a known manner.

The forms of the preparation of the composition for administration, into which the composition of this invention is formulated, may be any of the usual preparations of the composition for oral administration, injection or suppository, but the preparations for oral administration are more desirable. In formulating the preparations for oral administration, siastatin B or the siastatin B derivatives of the general formula (I) as the active ingredient for this invention may be mixed with a solid carrier or carriers and may further be added, if necessary, with excipient, binder, disintegrator, lubricant, colorant, flavoring agent, smell improver, surfactant and the like, and then the resulting mixture may be formulated into the form of tablets, granules, powder, capsules, pills, etc in a conventional manner. Siastatin B or the siastatin B derivatives of the general formula (I) may be incorporated, in the composition or preparation of this invention, in a proportion of 0.1-100 % by weight, preferably 1-90 % by weight.

The administered dose of the compound of the general formula (I) contained in the composition of this invention may vary dependently upon the age, body weight, sex, symptom of disease, purpose of therapeutic treatment and other factors and is to be decided by judgment of experts. In general, preferred dose of the compound of the general formula (I) as active ingredient is 50-500 mg per day for parenteral administrations. While, for oral administrations, a dose of the compound of 100-1000 mg per day is preferred. The administration may be done once a day or in 2-4 portions per day.

The pharmaceutical composition according to the first aspect of this invention may be administered in such case when the diarrhea to be prevented or treated is the one as induced in association with the administration of an anticancer chemotherapeutic agent, particularly in such a case when the diarrhea to be prevented or treated is the one as induced in association with the administration of the anticancer chemotherapeutic agent of such type that administered anticancer agent is converted in vivo into the glucuronides of the metabolic products and said glucuronides are then excreted into the bile.

Further, the pharmaceutical composition according to the first aspect of this invention may be administered in such case when the diarrhea to be prevented or treated is the one as induced in association with the administration of a topoisomerase I inhibitor as the anticancer chemotherapeutic agent, and particularly in such case when the diarrhea to be prevented or treated is the one induced in association with an intravenous administration of an anticancer camptothecin derivative, particularly irinotecan or topotecan, which acts as the topoisomerase I inhibitor.

According to a second aspect of this invention, there is further provided a use of siastatin B or a siastatin B derivative of the general formula (I) defined hereinbefore or a pharmacologically acceptable salt thereof, for the manufacture of a pharmaceutical composition to be used for the prevention or treatment of a diarrhea.

According to a third aspect of this invention, there is also provided a therapeutic method for preventing or treating a diarrhea as induced in a cancer-bearing patient, which comprises orally administering siastatin B or a siastatin B derivative of the general formula (I) as above, to the cancer-bearing patient having a symptom of the diarrhea or susceptible of causing the diarrhea due to the administration of such an anticancer camptothecin derivative acting as a topoisomerase I inhibitor, with siastatin B or the siastatin B derivative being administered at a dose effective and sufficient to ameliorate the symptom of the diarrhea.

In the method according to the third aspect of this invention, siastatin B or the siastatin B derivative of general formula (I) may be orally administered to the cancer-bearing patient having a symptom of diarrhea as induced by an intravenous administration of irinotecan or other anticancer camptothecin derivative as an anticancer agent, in such way that the administration of siastatin B or the siastatin B derivative is made prior to each time of intravenous injections of irinotecan or the other anticancer camptothecin derivative at a dose of siastatin B or the siastatin B derivative which is effective to ameliorate the symptom of diarrhea. As the administered dose for ameliorating the symptom of diarrhea, the compound of the general formula (I) may be orally administered to an adult at its dose of 100-1000 mg once per day. When an anticancer camptothecin derivative, particularly irinotecan or topotecan, is injected intravenously to the patient, it is usually preferred that the compound of the general formula (I) is administered orally at a time point of 10-30 minutes prior to each time of said intravenous injection.

## BEST MODE FOR CARRYING OUT THE INVENTION

This invention is now further illustrated with reference to Examples which show some preparations of the pharmaceutical composition of this invention, as well as Test Examples which show some therapeutic treatments of diarrhea. However, this invention is in no way limited to these Examples.

### Example 1

A powder preparation of the following composition was formulated by mixing well the under-mentioned ingredients in a conventional manner.

| | |
|---|---|
| 6-Deacetamido-6-trifluoroacetamido-siastatin B of the formula (Ib) | 100 mg |
| Lactose | 175 mg |

### Example 2

A capsule preparation was formulated by filling up a powder composition consisting of a mixture of the under-mentioned ingredients in capsule containers.

| | |
|---|---|
| Siastatin B derivative of the formula (Ib) | 100 mg |
| Lactose | 175 mg |

### Test Example 1

The inhibitory effect of the siastatin B derivative against a diarrhea as induced by administration of irinotecan was tested by the following procedure.

To each of male ICR mice of six weeks-aged (six mice per group), was administered intravenously a solution of irinotecan (one of the camptothecin derivatives) dissolved in a physiological salt solution, at a dose of 60 mg/ kg once per day for the five consecutive days, to produce model mice having digestive tract disorder with the diarrhea as induced. Using said model mice, there was administered orally 6-deacetamido-6-trifluoro-acetamidosiastatin B of the formula (Ib) defined hereinbefore as the test drug at a dose of 200 mg/kg or 400 mg/kg once per day for the five consecutive days, provided that each administration of the test drug was done at a time point of 15 minutes before each time of the administration of irinotecan. Change in the body weight of mice in each group under test was measured, and the test results of the measurements are given in Table 1 below.

Table 1

| Groups of experimental mice | Body weight change in each group of mice (6 mice per group)(average body weight:(g) ±SD) | | |
|---|---|---|---|
| | First day of the irinotecan administration | Terminal day of the irinotecan administration | 2nd Day after the terminal day of the irinotecan administration |
| Group untreated | 30.8 ± 3.0 | 31.2 + 2.6 | 31.6 ± 1.8 |
| Group treated by administration of irinotecan alone | 31.3 ± 4.3 | 27.9 ± 3.3 | 24.8 ± 1.5 |
| Group treated by administration of both of irinotecan + the test drug (at dose of 200 mg/kg) | 30.4 ± 0.5 | 28.3 ± 2.3 | 26.2 ± 3.1 |
| Group treated by administration of both of irinotecan + the test drug (at dose of 400 mg/kg) | 31.3 ± 3.9 | 29.9 ± 2.8 | 27.1 ± 3.7 |

Further, in order to evaluate the extent of the change in the body weight of mice in each group (6 mice per group) above-mentioned, the relative ratio of the body weight was calculated according to a calculation equation.
The calculation equation is as follows.

Relative ratio of body weight = (the body weight as measured at the day of measurement) ÷ (the body weight as measured at the first day of the administration of irinotecan)

The test results so obtained are given in Table 2 below.

Table 2

| Groups of experimental mice | Relative ratio of body weight | |
|---|---|---|
| | First day of the irinotecan administration | 2nd Day after the terminal day of the irinotecan administration |
| Group untreated | 1.02 ± 0.02 | 1.03 ± 0.02 |
| Group treated by administration of irinotecan alone | 0.89 ± 0.04 | 0.80 ± 0.06 |
| Group treated by administration of both of irinotecan + the test drug (at dose of 200 mg/kg) | 0.93 ± 0.04 | 0.86 ± 0.02 |
| Group treated by administration of both of irinotecan + the test drug (at dose of 400 mg/kg) | 0.96 + 0.05 | 0.87 ± 0.06 |

As shown in Table 1 and Table 2, the siastatin B derivative used as the test drug could significantly reduce the loss of the body weight which would be induced by the administration of irinotecan.
Further, in order to estimate the extent of the disorders in the digestive tracts of mice under test which were

induced at the second day after the terminal day of the administration of irinotecan (that is, just at the second day after the terminal day of the administration of 6-deacetamido -6-trifluoroacetamido-siastatin B as the test drug), the observations of the extent of diarrhea as well as the pathohistological observations of tissues of the lower small intestine were evaluated. The results of evaluation so obtained are shown in Table 3 below.

Table 3

| Group of experimental mice | Extent of diarrhea and pathohistological observations of digestive tract | | | | | | |
|---|---|---|---|---|---|---|---|
| | Number of mice on tested | Observations the extent of diarrhea | | | | pathohistological observation of lower small intestine | |
| | | - | + | ++ | +++ | Necrosis of mucosa | Submucous edema |
| Group untreated | 6 | 6 | 0 | 0 | 0 | - | - |
| Group treated by administration of irinotecan alone | 6 | 0 | 1 | 1 | 4 | +++ | +++ |
| Group treated by administration of irinotecan + the test drug (at dose of 200 mg/ kg) | 6 | 4 | 1 | 1 | 0 | +/++ | +/++ |
| Group treated by administration of irinotecan + the test drug (at dose of 400mg/ kg) | 6 | 4 | 2 | 0 | 0 | +/++ | + |

The observations of the extent of diarrhea was based upon the following scales.

- : normal, + : slight,
++ : moderate, +++ : severe.

As be revealed from Table 3 above, it is clear that the tests with the combined administration of irinotecan and the siastatin B derivative as the test drug were able to lead that the extent of diarrhea as revealed from the observations of the symptom of diarrhea, as well as the extent of the disorders of the digestive tract as revealed from the pathohistological observations of the small intestine were ameliorated, as compared with those which were found in the comparative group of mice having received the administration of irinotecan alone.

Test Example 2

The following test was conducted to verify any influence on the anticancer efficacy of irinotecan, by the combined administration of the siastatin B derivative as test drug, with irinotecan.

To the cecum of each of nude mice (female) of KSN strain and of eight weeks aged (7-10 mice per group), under anesthetization, was transplanted a tumor fragment of human colon cancer strain HT-29, thereby to produce model mice with colon cancer. At the last day of the third week from the transplantation of the tumor fragment, irinotecan was administered to said model mice by the intravenous injection once per four days, totally five times, so that 60 mg/ kg of irinitecan was administered at each of the first and second injections and 45 mg/kg of irinitecan was administered at each of the third, fourth and fifth injections. The test drug, 6-deacetamido-6-trifluoroacetamido-siastatin B of the

formula (Ib), was administered to each of these model mice at a dose of 400 mg/kg, once per four days, totally five times, so that each administration of the test drug was made at a time point of 15 minutes prior to each of the intravenous administrations of irinotecan. There was further provided another group of mice (comparative) to which was administered said test drug alone at a dose of 400 mg/kg.

Two days after the terminal day of the administration of the test drug, the mice of each group under test were sacrificed under anesthetization, and the tumor masses were removed from the cecum sites. The tumor mass as removed from each of the mice was weighed. The results obtained are shown in Table 4 below. The influence of the tested siastatin B derivative which affected upon the medicinal efficacy of irinotecan was estimated with reference to the change in the weight of tumor mass as an index.

Table 4

| Group of experimental mice | Change in weight of tumor mass of colon cancer HT-29 as transplanted to cecum | |
|---|---|---|
| | Number of mice under test | Weight of tumor mass(mg) |
| Group untreated | 10 | 70.0 + 21.2 |
| Group administrated with the test drug alone | 10 | 63.0 ± 13.4 |
| Group administrated with irinotecan alone | 10 | 38.3 ± 6.4* |
| Group administrated with irinotecan + the test drug | 7 | 39.0 ± 7.4* |
| The asterisk mark * indicates that the data are admitted to have a significant deviation (P<0.01) over those of the untreated group. | | |

As shown in Table 4 above, it is demonstrated that the tumor weight in the group of mice which were treated by the administration of irinotecan alone was reduced lower than the tumor weight in the untreated group (which received neither of irinotecan and the siastatin B derivative as the test drug), indicating that irinotecan could exhibit a significant inhibitory effect against the growth of the cells of human colon cancer strain HT-29 as transplanted to the cecum of nude mouse under test.

Further, as shown in Table 4 above, it is clear that the tumor weight in the comparative group of mice as treated by administration of irinotecan alone was substantially equal to the tumor weight in the treated group of mice which were treated by the combined administration of both of irinotecan and the test drug (namely, the treated group of mice as administered with irinotecan + the siastatin B derivative). In view of this fact, it is evident that the siastatin B derivative administered as the test drug did not exhibit any inhibitory effect against the anticancer activity of irinotecan itself.

## INDUSTRIAL APPLICABILITY

As explained hereinbefore, siastatin B or the siastatin B derivatives of the general formula (I), which is usable as an active ingredient in the pharmaceutical composition of this invention for prevention or treatment of a diarrhea, has an inhibitory activity against β-glucuronidase. The composition of this invention, which comprises as active ingredient siastatin B or the siastatin B derivative of general formula (I), when orally administered to cancer-bearing patients, is able to well inhibit a diarrhea as induced in association with the administration of an anticancer agent to the patients, and particularly the composition of this invention is able to inhibit such a diarrhea as induced in association with the administration of a camptothecin derivative as anticancer agent. Therefore, the composition of this invention can eliminate a necessity to discontinue the chemotherapy of cancer which would often becomes necessary due to an incurred diarrhea. Thus, the composition of this invention makes the continuance of such cancer-chemotherapy possible and can also prevent the patients from exhaustion of their physical strength which would be involved due to the diarrhea. Thus, the composition and method according to this invention can further improve the therapeutic effect which is attainable by the anticancer agents known and used in the prior art.

## Claims

**1.** A pharmaceutical composition for preventing or treating a diarrhea, which comprises as an active ingredient, siastatin B or a siastatin B derivative that has an inhibitory activity against β-glucuronidase and that is represented by the following general formula (I)

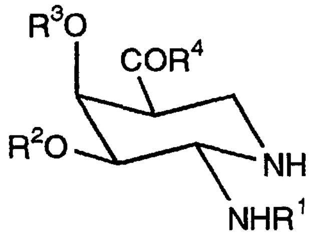

(I)

wherein $R^1$ is a hydrogen atom or an unsubstituted or halogen-substituted, straight or branched acyl group of 1-5 carbon atoms, particularly an alkanoyl group, or $R^1$ is a group of formula -C(=NH)$R^5$ in which $R^5$ is a group -$OR^6$ or $R^5$ is a group -$NR^7$ $R^8$ where $R^6$, $R^7$ and $R^8$ each stand for a hydrogen atom or a straight or branched alkyl group of 1-5 carbon atoms; and $R^2$ and $R^3$ may be the same or different from each other and $R^2$ and $R^3$ each are a hydrogen atom or an unsubstituted or halogen-substituted, straight or branched acyl group of 1-5 carbon atoms, particularly an alkanoyl group; and independently from $R^5$, $R^4$ stands for a group of formula -$OR^6$ or a group of formula -$NR^7$ $R^8$ where $R^6$, $R^7$ and $R^8$ each stand for a hydrogen atom or a straight or branched alkyl group of 1-5 carbon atoms, or a pharmacologically acceptable salt thereof, in combination with a pharmacologically acceptable carrier or carriers.

**2.** A composition as claimed in Claim 1, which comprises as the active ingredient, a siastatin B derivative of the general formula (I) where $R^1$, $R^2$ and/or $R^3$ is or each are an unsubstituted or halogen-substituted, straight or branched ($C_1$-$C_5$) alkanoyl group, or a salt thereof.

**3.** A composition as claimed in Claim 2, which comprises as the active ingredient, a siastatin B derivative of the general formula (I) wherein $R^1$, $R^2$ and/or $R^3$ is or each are an unsubstituted or halogen-substituted, straight or branched ($C_1$-$C_5$) alkanoyl group that is chosen from formyl group, acetyl group, propionyl group, butyryl group, isobutyryl group, valeryl group, isovaleryl group, 2-methylbutyryl group, 2,2-dimethylpropionyl group, fluoroacetyl group, difluoroacetyl group, trifluoroacetyl group, chloroacetyl group, dichloroacetyl group, trichloroacetyl group, bromoacetyl group, iodoacetyl group, pentafluoropropionyl group, pentachloropropionyl group, heptafluorobutyryl group and heptachloroisobutyryl group, or a salt thereof.

**4.** A composition as claimed in Claim 1, which comprises as the active ingredient, a compound of the general formula (I) wherein $R^1$ is hydrogen atom, acetyl group, trifluoroacetyl group, trichloroacetyl group or amidino group -C(=NH)$NH_2$; and $R^2$ and $R^3$ each are hydrogen atom; and $R^4$ is a group -OH, or a salt thereof.

**5.** A composition as claimed in Claim 1, in which siastatin B or a siastatin B derivative of the general formula (I) contained therein is a siastatin B derivative of the general formula (I')

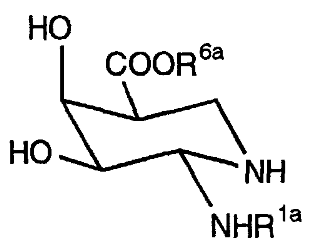

(I')

wherein $R^{1a}$ is hydrogen atom or a ($C_1$-$C_5$) alkanoyl group, or $R^{1a}$ is a ($C_1$-$C_5$) alkanoyl group which is mono-, di- or trisubstituted with chlorine or fluorine atom, or $R^{1a}$ is amidino group of formula -C(=NH)$NH_2$; and $R^{6a}$ is hydrogen atom or a ($C_1$-$C_5$) alkyl group, or a pharmacologically acceptable salt thereof.

**6.** A composition as claimed in Claim 1, which comprises as the active ingredient, siastatin B of the formula (Ia)

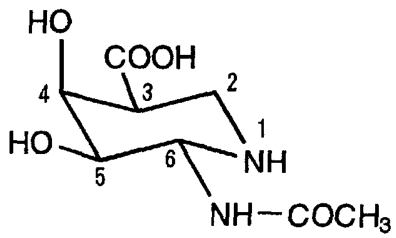

(Ia)

or 6-deacetamido-6-trifluoroacetamido-siastatin B of the formula (Ib)

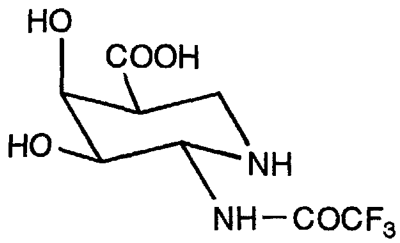

(Ib)

or 6-deacetamido-6-trichloroacetamido-siastatin B of the formula (Ic)

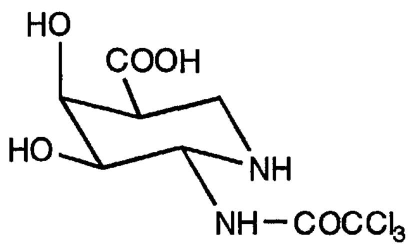

(Ic)

or 6-deacetamido-6-guanidino-siastatin B of the formula (Id)

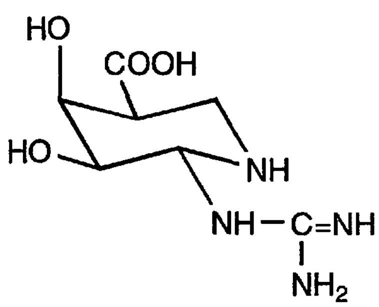

(Id)

or 6-deacetamido-6-amino-siastatin B of the formula (Ie)

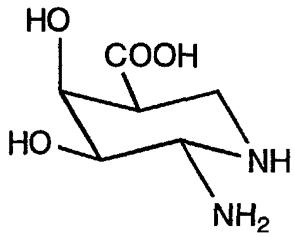

(Ie)

or a pharmacologically acceptable salt thereof.

**7.** A composition as claimed in any one of Claims 1-6, which is used so that the diarrhea to be prevented or treated is a diarrhea as induced by the administration of an anticancer chemotherapeutic agent.

**8.** A composition as claimed in any one of Claims 1-7, which is used so that the diarrhea to be prevented or treated is a diarrhea as induced by the administration of an anticancer chemotherapeutic agent of such type that a metabolic product of the administered anticancer agent can be converted into the glucuronide thereof which is then excreted into the bile.

**9.** A composition as claimed in Claim 7 or 8, which is used so that the diarrhea to be prevented or treated is a diarrhea as induced by the administration of a topoisomerase I inhibitor acting as the anticancer chemotherapeutic agent.

**10.** A composition as claimed in Claim 8 or 9, which is used so that the diarrhea to be prevented or treated is a diarrhea as induced by the intravenous administration of an anticancer camptothecin derivative, particularly irinotecan or topotecan, that is a topoisomerase I inhibitors.

**11.** Use of siastatin B or a siastatin B derivative of the general formula (I) or a pharmacologically acceptable salt thereof as defined in Claim 1, for the manufacture of a pharmaceutical composition to be used for the prevention or treatment of a diarrhea.

**12.** A method for the prevention or therapeutic treatment of a diarrhea as induced in a cancer-bearing patient, which comprises administering orally siastatin B or a siastatin B derivative of the general formula (I) as defined in Claim 1, to the patient having a symptom of the diarrhea or susceptible of causing the diarrhea due to the administration of such an anticancer camptothecin derivative acting as a topoisomerase I inhibitor, with siastatin B or the siastatin B derivative being administered at a dose effective and sufficient to ameliorate the symptom of the diarrhea.

**13.** A method as claimed in Claim 12, wherein siastatin B or a siastatin B derivative of the general formula (I) as defined in Claim 1 is orally administered to a cancer-bearing patient having a symptom of diarrhea as induced by intravenous administration of irinotecan or the other anticancer camptothecin derivative as an anticancer agent, in such way that the administration of siastatin B or the siastatin B derivative is made prior to each time of the intravenous administrations of irinotecan or the other anticancer camptothecin derivative, at a dose of siastatin B or the siastatin B derivative effective to ameliorate the symptom of diarrhea.

## INTERNATIONAL SEARCH REPORT

International application No.
PCT/JP00/04264

A. CLASSIFICATION OF SUBJECT MATTER
Int.Cl$^7$ A61K31/7012, A61P1/12 // C07H7/02

According to International Patent Classification (IPC) or to both national classification and IPC

B. FIELDS SEARCHED

Minimum documentation searched (classification system followed by classification symbols)
Int.Cl$^7$ A61K31/7012, A61P1/12, C07H7/02

Documentation searched other than minimum documentation to the extent that such documents are included in the fields searched

Electronic data base consulted during the international search (name of data base and, where practicable, search terms used)
CAPLUS(STN),CAOLD(STN),REGISTRY(STN)

C. DOCUMENTS CONSIDERED TO BE RELEVANT

| Category* | Citation of document, with indication, where appropriate, of the relevant passages | Relevant to claim No. |
|---|---|---|
| Y | TAKAHIKO SATOH et al., "Synthesis and Antimetastatic Activity of 6-Trichloroacetamido and 6-Guanidino Analogues of Siastatin B", THE JOURNAL OF ANTIBIOTICS, Vol.49, No.3 (MAR.1996) p.321-325 | 1-11 |
| Y | YOSHIO NISHIMURA et al., "TOTALLY SYNTHETIC ANALOGUES OF SIASTATIN B III.TRIFLUOROACETAMIDE ANALOGUES HAVING INHIBITORY ACTIVITY FOR TUMOR METASTASIS", THE JOURNAL OF ANTIBIOTICS, Vol.47, No.1 (JAN.1994) p.101-107 | 1-11 |
| Y | KIYOSHI TAKASUNA,et al., "Protective Effects of *Kampo* Medicines and Baicalin against Intestinal Toxicity of a New Anticancer Camptothecin Derivative,Irinotecan Hydrochloride(CPT-11), in Rats", Jpn. J. Cancer Res., Vol.86 (October 1995) p.978-984 | 1-11 |
| Y | KIYOSHI TAKASUNA et al., "Involvement of β-Glucuronidase in Intestinal Microflora in the Intestinal Toxicity of the Antitumor Camptothecin Derivative Irinotecan Hydrochloride (CPT-11) in Rats" | 1-11 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 September, 2000 (18.09.00) | 26 September, 2000 (26.09.00) |
| Name and mailing address of the ISA/ Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP00/04264

**Box I Observations where certain claims were found unsearchable (Continuation of item 1 of first sheet)**

This international search report has not been established in respect of certain claims under Article 17(2)(a) for the following reasons:

1. ☒ Claims Nos.: 12,13
because they relate to subject matter not required to be searched by this Authority, namely:
Claims 12 and 13 pertain to methods for treatment of the human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)